# DEMANDE DE BREVET EUROPEEN

(11) **EP 2 511 256 A1**
(43) Date de publication de la demande: **17.10.2012**
(21) Numéro de dépôt: 12163613.8
(22) Date de dépôt: 10.04.2012
(51) Int. Cl.: C07C 67/54, C07C 67/62, C07C 69/80, C07C 67/08, B01D 17/04

(54) **Procede de fabrication de phtalate de dialkyle**

(30) Priorité: 12.04.2011 FR 1153182; 15.09.2011 FR 1158221
(71) Demandeur: ARKEMA FRANCE, 92700 Colombes (FR)
(72) Inventeur: Paul, Jean-Michel, 57070 METZ (FR); Levray, André, 57890 Porcelette (FR); Riondel, Alain, 57600 FORBACH (FR)

(57) **Abrégé**

La présente invention concerne la fabrication de phtalates de dialkyle par estérification de l'anhydride phtalique par des alcools aliphatiques de longueur de chaîne comportant plus de 8 atomes de carbone.

Plus particulièrement, l'invention a pour objet un procédé performant permettant de produire du phtalate de dialkyle par réaction d'anhydride phtalique et d'un alcool aliphatique de longueur de chaîne comportant de 9 à 13 atomes de carbone, en évitant la formation d'émulsion au niveau de la distillation finale du solvant dans le cas d'une catalyse sulfurique en milieu solvant tel que le cyclohexane. Selon l'invention, un additif choisi parmi les polymères cationiques hydrosolubles, les alcools polyvinyliques, les polychlorures d'aluminium ou le sulfate de magnésium anhydre ou hydraté est ajouté dans la phase organique décantée après neutralisation du mélange réactionnel issu de l'estérification.

## Description

La présente invention concerne la fabrication de phtalates de dialkyle par estérification de l'anhydride phtalique par des alcools aliphatiques de longueur de chaîne comportant plus de 8 atomes de carbone.

Les esters dérivés d'acides monocarboxyliques ou polycarboxyliques aromatiques ou aliphatiques tels que les acides phtalique, adipique, sébacique, trimétillique, et d'alcools aliphatiques tels que les hexanols, le 2-éthyl hexanol, le n-octanol, les décanols, les tridécanols, ou encore de polyols aliphatiques, sont connus pour leurs utilisations comme plastifiants de diverses matières plastiques, en particulier du PVC. En général, les plastifiants rendent les matières plastiques souples, flexibles, ils améliorent leur résistance aux chocs et au froid, l'allongement à la rupture et ils facilitent leur mise en oeuvre. Les plastifiants sont de ce fait présents dans de nombreuses matières plastiques souples et on les retrouve dans de nombreux produits industriels et produits de consommation tels que, par exemple, les produits pour automobile, les isolants pour câbles et fils souples, les revêtements pour planchers et murs, les produits de grande consommation en plastique, les jouets, les cosmétiques et produits de soin personnel, le matériel médical, les emballages de denrées alimentaires.

Parmi les composés utilisables comme plastifiants, les diesters de l'acide phtalique, appelés aussi phtalates de dialkyle, représentent la plus grande part.

Toutefois, les phtalates les plus répandus, dont en particulier le phtalate de 2-éthyl hexyle connu aussi sous les dénominations DEHP ou DOP (dioctyle phtalate) font l'objet d'études par divers organismes afin de clarifier certaines questions en suspens quant à la toxicité de ces composés et aux normes à appliquer.

Par conséquent, il existe un réel besoin de trouver des produits de substitution au DEHP, présentant une innocuité pour la santé humaine et vis-à-vis de l'environnement, mais en gardant les mêmes propriétés de résistance et de flexibilité, notamment pour le secteur médical et pour les emballages alimentaires. Une des voies envisagées consiste à utiliser des phtalates de plus haut poids moléculaire, en particulier des phtalates dérivés d'alcools de longueur de chaine supérieure à celle du 2-éthyl hexanol.

La présente invention a donc pour objet la synthèse de phtalates de dialkyle par estérification de l'anhydride phtalique par des alcools aliphatiques ROH de longueur de chaîne comportant plus de 8 atomes de carbone, l'estérification étant catalysée par l'acide sulfurique.

Le mécanisme réactionnel peut être schématisé par les 2 réactions suivantes :
1) formation de phtalate acide de monoalkyle 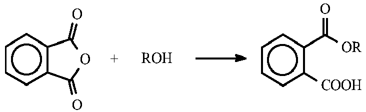
2) formation du phtalate de dialkyle

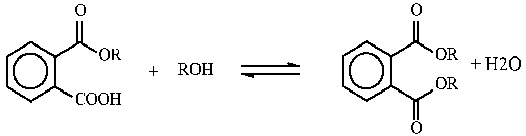

La réaction se fait en léger excès d'alcool de façon à obtenir une conversion totale de l'anhydride phtalique, en général dans un solvant organique formant un azéotrope avec l'eau, l'eau générée lors de la réaction étant alors éliminée au fur et à mesure par distillation sous forme d'azéotrope. Il s'ensuit une série de traitements, neutralisation, séparation des phases aqueuse et organique, lavages à l'eau, évaporation du solvant, séchage, permettant d'isoler le phtalate de dialkyle recherché avec une pureté analytique d'au moins 99,5%.

Dans le cas d'une catalyse à l'acide sulfurique, le mélange réactionnel obtenu à l'issu de la réaction, contient outre le phtalate de dialkyle recherché, des sous-produits résiduels (anhydride phtalique PAA, alcool ROH, phtalate de monoalkyle), de l'eau, du solvant, mais aussi du sulfate acide d'alkyle RHSO₄ et du sulfate de dialkyle R₂SO₄, résultant de l'estérification partielle de l'acide sulfurique par l'alcool ROH.

Une étape de neutralisation avec un agent alcalin, habituellement l'hydroxyde de sodium ou le carbonate de sodium, permet de neutraliser le sulfate acide d'alkyle sous la forme de sulfate mixte d'alkyle et de métal alcalin (sodium) RNaSO₄, qui est éliminé lors des différents lavages à l'eau.

Il en résulte une phase organique, qui, après décantation, est exempte de sulfate acide d'alkyle, sous forme acide RHSO₄ ou sous forme neutralisée RNaSO₄; cette phase est ensuite purifiée en plusieurs étapes, la première d'entre elles consistant à éliminer d'une part le solvant utilisé à la réaction pour éliminer l'eau, généralement le cyclohexane, sous la forme d'un hétéroazéotrope cyclohexane/eau, et d'autre part l'eau. Les étapes suivantes de purification conduisent à l'obtention du phtalate de dialkyle pur recherché.

Dans le procédé de fabrication d'esters d'anhydride phtalique en présence d'acide sulfurique décrit dans le document US 3,033,895, la présence résiduelle d'esters gras dans les alcools utilisés pour la production de ces diesters génère la formation d'émulsions difficiles à séparer. Ces problèmes d'émulsion ont pu être résolus par une étape de neutralisation du mélange brut réaction réalisée dans des conditions spécifiques de concentration et de température.

De façon surprenante, il a maintenant été constaté que la présence résiduelle de sulfate acide d'alkyle (sous forme acide RHSO₄ ou sous forme neutralisée RNaSO₄), dans la phase organique décantée, entraîne la formation d'une émulsion stable au cours de l'étape d'élimination du cyclohexane et de l'eau, lorsqu'il s'agit de la fabrication de phtalate de dialkyle à partir d'alcools aliphatiques ROH de longueur de chaîne comportant plus de 8 atomes de carbone, alors que ce problème n'apparaît pas dans le cas de la fabrication du phtalate de 2-éthyl hexyle, ni plus généralement dans le cas de la fabrication des phtalates de dialkyle comportant jusqu'à 8 atomes de carbone.

La présence résiduelle de sulfate acide d'alkyle sous forme acide RHSO₄ dans la phase organique décantée peut être due à une neutralisation insuffisante du mélange réactionnel, le sulfate acide d'alkyle RHSO₄ formé au cours de la réaction restant alors dans la phase organique même après lavage à l'eau et décantation.

La présence résiduelle de sulfate acide d'alkyle sous forme neutralisée RNaSO₄ dans la phase organique décantée peut être liée à une mauvaise décantation de la phase aqueuse contenant RNaSO₄ après l'étape de neutralisation, laissant un peu de RNaSO₄ dans la phase organique.

Dans ces deux cas, mauvaise neutralisation du mélange réactionnel et/ou mauvaise décantation après neutralisation, il y a formation d'une émulsion présentant un aspect crémeux qui perturbe complètement le fonctionnement de l'étape de distillation du cyclohexane et de l'eau effectuée sur la phase organique décantée contenant le phtalate de dialkyle recherché, de longueur de chaîne comportant plus de 8 atomes de carbone. Cette émulsion qui se forme même pour des taux minimes de sulfate acide d'alkyle (sous forme acide RHSO₄ ou sous forme neutralisée RNaSO₄), est stable durant plusieurs semaines, elle peut être cassée par centrifugation, mais cela nécessite d'arrêter l'atelier, avec pour conséquence une baisse de la productivité de l'installation industrielle.

Il existe donc un réel besoin d'éviter la formation d'émulsion au niveau de la distillation finale du solvant dans un procédé de fabrication de phtalate de dialkyle par estérification de l'anhydride phtalique par des alcools aliphatiques ROH de longueur de chaîne comportant plus de 8 atomes de carbone, l'estérification étant effectuée en milieu solvant tel que le cyclohexane et catalysée par l'acide sulfurique. Dans le cas où ladite émulsion se formerait, il est nécessaire de disposer également d'un moyen évitant l'arrêt total de l'installation.

Un objet de la présente invention est donc de fournir un procédé performant permettant de produire du phtalate de dialkyle par réaction d'anhydride phtalique et d'un alcool aliphatique de longueur de chaîne comportant plus de 8 atomes de carbone et ne présentant pas les inconvénients précités dans le cas d'une catalyse sulfurique en milieu solvant tel que le cyclohexane.

La présente invention a encore pour but de fournir un procédé modulable et flexible, c'est-à-dire réglable facilement et rapidement en fonction des incidents de production rencontrés, et qui s'adapte aisément aux dispositifs de fabrication existants dans l'industrie des phtalates.

La Société Déposante a trouvé que l'utilisation à très faible teneur d'additifs particuliers permet d'éviter l'apparition d'une émulsion stable en cas de mise en contact sous agitation de sulfate acide d'alkyle (sous forme acide RHSO₄ ou sous forme neutralisée RNaSO₄, R contenant plus de 8 atomes de Carbone) avec un mélange cyclohexane /eau.

La Société Déposante a également trouvé qu'il est possible de casser l'émulsion, une fois celle-ci formée, à l'aide de ces additifs.

Compte tenu du contexte technique des ateliers de phtalates avec une catalyse sulfurique, ces additifs doivent répondre à un certain nombre d'exigences :
- être solubles dans l'eau afin de pouvoir être éliminés par lavage à l'eau et ne pas, ainsi, se retrouver dans le phtalate de dialkyle final.
- ne pas colorer le phtalate de dialkyle recherché.
- ne pas générer de dysfonctionnement au niveau du traitement final des eaux produites par l'atelier.
- être efficaces à très faible teneur (quelques dizaines de ppm).
- être compatibles avec l'utilisation finale du phtalate de dialkyle comme plastifiant du PVC.

Dans le document WO 2010/057847 relatif à un procédé de fabrication de phtalates de dialkyle à longue chaine, un polymère cationique hydrosoluble est ajouté dans la phase organique obtenue après neutralisation du milieu réactionnel et décantation. Cependant, ce procédé n'utilise pas une catalyse à l'acide sulfurique, et ne génère donc pas de sous-produits à base de sulfates d'alkyle à l'origine du problème d'émulsion qu'entend résoudre la présente invention.

La Société Déposante a trouvé que les additifs particulièrement adaptés peuvent être choisis parmi les polymères cationiques hydrosolubles les alcools polyvinyliques de préférence partiellement hydrolysés avec un taux d'hydrolyse allant notamment de 38 à 99%, de préférence de 70 à 90%, les polychlorures d'aluminium, et le sulfate de magnésium anhydre ou hydraté.

Dans le cas des polymères cationiques hydrosolubles, la Société Déposante a trouvé d'une façon surprenante, que ces additifs bien que connus pour déstabiliser le PVC, n'altéraient pas les propriétés mécaniques et thermiques du PVC plastifié avec du phtalate de dialkyle, en particulier avec du DPHP, obtenu suivant un procédé utilisant ce type d'additif.

La présente invention a donc pour objet un procédé de fabrication de phtalate de dialkyle par estérification d'anhydride phtalique avec un alcool ROH, R représentant un groupe alkyle linéaire ou ramifié comportant de 9 à 13 atomes de carbone, en présence d'acide sulfurique et d'un solvant formant un azéotrope avec l'eau, **caractérisé en que** l'on ajoute au moins un additif choisi parmi les polymères cationiques hydrosolubles, les alcools polyvinyliques de préférence partiellement hydrolysés avec un taux d'hydrolyse allant notamment de 38 à 99%, de préférence de 70 à 90%, les polychlorures d'aluminium, ou le sulfate de magnésium anhydre ou hydraté, en particulier le sulfate de magnésium cristallisé avec 7 molécules d'eau (dénommé dans la suite MgSO₄, 7H₂O).
soit dans la phase organique décantée après neutralisation du mélange réactionnel issu de l'estérification,
soit en tête de la colonne de distillation finale du solvant présent dans la phase organique décantée après neutralisation du mélange réactionnel issu de l'estérification.

Plus précisément, la présente invention a pour objet un procédé de fabrication de phtalate de dialkyle par estérification d'anhydride phtalique avec un alcool ROH, R représentant un groupe alkyle linéaire ou ramifié comportant de 9 à 13 atomes de carbone, comprenant les étapes suivantes :
a) on met à réagir un alcool ROH avec de l'anhydride phtalique dans un rapport molaire alcool / anhydride allant de 2,1 à 2,4, à une température allant de 120°C à 140°C, en l'absence de catalyseur, jusqu'à l'obtention d'une phase homogène limpide,
b) on ajoute à chaud de l'acide sulfurique à une teneur comprise entre 0,2% à 0,9% en poids (exprimé en H₂SO₄ 100%), par rapport à l'anhydride phtalique, et un solvant organique, la température de réaction étant maintenue entre 120°C et 140°C et l'eau étant éliminée en continu par distillation sous forme d'un hétéroazéotrope avec le solvant, durant toute la durée de la réaction,
c) on neutralise le mélange brut réactionnel à l'aide d'un agent alcalin à une température comprise entre 50°C et 70°C, et on sépare, après décantation, une phase organique et une phase aqueuse,
d) on distille le solvant de ladite phase organique,
e) on effectue un traitement thermique de la phase organique obtenue à l'étape d), puis différents cycles de lavages à l'eau/décantation, et une distillation finale pour obtenir le phtalate de dialkyle pur recherché,
   **caractérisé en que** l'on ajoute au moins un additif choisi parmi les polymères cationiques hydrosolubles, les alcools polyvinyliques de préférence partiellement hydrolysés avec un taux d'hydrolyse allant notamment de 38 à 99%, de préférence de 70 à 90%, les polychlorures d'aluminium ou le sulfate de magnésium anhydre ou hydraté, soit à l'étape c) dans la phase organique après décantation, soit au cours de l'étape d) en tête de la colonne de distillation.

D'autres caractéristiques et avantages de l'invention ressortiront mieux à la lecture de la description détaillée qui suit et des exemples de réalisation non limitatifs de l'invention, en référence à la figure unique annexée qui représente un schéma de l'installation pour mettre en oeuvre le procédé selon l'invention.

Selon l'invention, les additifs utilisables sont par exemple, des polymères cationiques dérivés de l'epichlorhydrine et de la dimethyl amine, tels que ceux commercialisés par la société CECA sous la marque PROCHINOR^{®}, ou des alcools polyvinyliques avec un taux d'hydrolyse de 38 à 99%, tels que ceux commercialisés par Kuraray, sous la marque POVAL^{®}, des polychlorures d'aluminium par exemple ceux commercialisés sous le nom FLOCAT par la société SNF FLOERGER.

Comme sulfate de magnésium anhydre ou cristallisé avec 7 molécules d'eau, on peut utiliser les produits commercialisés par les sociétés Brenntag et Stockmeier sous forme solide ou sous forme de solution aqueuse à 20% en poids.

On peut citer de manière non limitative, les produits commerciaux suivants : PROCHINOR^{®} FL185 ; FL7980 ; FL4809 ; PX4901 ; FL07 , les additifs commercialisés par NALCO, NALCO^{®} EC 6129A, AQUAMAX^{®} EC 3379 A, les additifs commercialisés par Kuraray POVAL^{®}, PVA 217SB, 205MB, 105, KL506, C506, MP203, LM 20, ou l'additif FLOQUAT PAC 18 de SNF FLOERGER.

L'additif est généralement ajouté à une teneur pouvant aller de 1 à 1000 ppm, de préférence de 5 à 750 ppm, de manière plus préférée de 5 à 500 ppm, ces teneurs étant exprimées par rapport à la phase organique décantée.

Les alcools utilisables dans le procédé selon l'invention sont des alcools ROH dont le sulfate acide d'alkyle RHSO₄ ou son sel de sodium RNaSO₄ présentent un caractère émulsionnant en présence d'un solvant tel que le cyclohexane et d'eau. Il s'agit de monoalcools à chaîne linéaire ou ramifiée, de longueur supérieure à celle du 2-éthyl hexanol, comportant de 9 à 13 atomes de carbone, de préférence comportant de 9 à 11 atomes de carbone, plus particulièrement 10 atomes de carbone, par exemple le décanol, le 2-propyl heptanol, le tridécanol, l'isononanol, l'isodécanol, le dodécanol, l'undécanol.

Le procédé selon l'invention est particulièrement bien adapté à la synthèse de phtalate de 2-propyl heptyle à partir de 2-propyl heptanol.

L'alcool 2-propyl heptanol peut être représenté sous la forme C₅H₁₁CH(C₃H₇)CH₂OH avec le groupe C₅H₁₁ pouvant représenter n-C₅H₁₁, C₂H₅CH(CH₃)CH₂ ou CH₃CH(CH₃)CH₂CH₂.

Par la dénomination simplifiée 2-PH, on entend le 2-propyl heptanol sous la forme de chacun de ces isomères seuls ou en mélange. Le 2-PH peut contenir en outre d'autres isomères en faible teneur, tels que 2-isopropyl heptanol, 2-isopropyl-4-méthyl hexanol, 2-isopropyl-5-méthyl hexanol ou 2-propyl-4,4-diméthyl pentanol.

Le 2-PH peut être produit de différentes manières, par exemple par aldolisation de n-valeraldéhyde produit par hydroformylation de butènes, déshydratation de l'alcool obtenu en 2-propyl-2-heptanal, suivie d'une hydrogénation. Le 2-PH peut être également obtenu par condensation de 1-pentanol (sous la forme d'un mélange de méthyl butanols) en présence de KOH à une température élevée selon une réaction de Guerbet.

Selon l'invention, le 2-PH contient majoritairement du 2-propyl heptanol. En général, le 2-PH est un mélange comprenant :
- de 70 à 99%, de préference de 80 à 95% de 2-propyl heptanol n-C₅H₁₁CH(C₃H₇)CH₂OH et
- de 1 à 30%, de préférence de 5 à 20% d'un mélange de 4-méthyl 2-propyl hexanol C₂H₅CH(CH₃)CH₂CH(C₃H₇)CH₂OH et de 5-méthyl 2-propyl hexanol CH₃CH(CH₃)CH₂CH₂CH(C₃H₇)CH₂OH

La première étape a) du procédé de l'invention est effectuée dans un réacteur 10 sans catalyseur en présence d'un excès molaire d'alcool (ROH), afin d'assurer une conversion totale de l'anhydride phtalique (PAA). Le rapport molaire ROH/ PAA est compris entre 2,1 et 2,4, préférentiellement entre 2,2 et 2,3. A la fin de la première étape qui peut durer d'une heure à deux heures, tout le PAA est converti majoritairement en phtalate acide d'alkyle et partiellement en phtalate de dialkyle.

Cette étape peut être effectuée en batch, par exemple dans un réacteur agité chauffé par double enveloppe ou circulation externe sur un bouilleur, ou en continu, par exemple dans un réacteur agité.

La seconde étape b) consistant à estérifier le phtalate acide d'alkyle en phtalate de dialkyle est réalisée dans un second réacteur 20 après introduction en 21 d'un solvant organique non aromatique formant un hétéroazéotrope avec l'eau et du catalyseur H₂SO₄. Parmi les solvants utilisables, on peut citer les hydrocarbures tels que le cyclohexane, l'heptane, etc. Avantageusement, la quantité de solvant introduite est comprise entre 100g et 300g, et de préférence entre 150g et 250 g pour 100g de PAA mis en oeuvre.

La seconde étape d'estérification est effectuée à une température comprise entre 120°C et 140°C, préférentiellement entre 125 et 135°C. L'eau générée par la réaction est éliminée au fur et à mesure par distillation azéotropique avec le solvant pendant toute la durée de la réaction qui est généralement comprise entre 4 et 6 heures.

Cette étape peut être effectuée en batch, par exemple dans un réacteur agité chauffé par double enveloppe ou circulation externe sur un bouilleur surmonté d'une colonne à distiller, ou en continu, par exemple dans un réacteur agité ou un réacteur type colonne de distillation réactive. La colonne à distiller 25 est pourvue d'un condenseur de tête et d'un décanteur 26 pour séparer l'eau de réaction 27 du solvant 22 qui peut être recyclé à la réaction.

L'étape suivante, l'étape c) du procédé selon l'invention, est la neutralisation du mélange brut réactionnel. Elle est réalisée dans un neutraliseur 30 à une température comprise entre 50°C et 70°C à l'aide d'un agent alcalin 31, classiquement une solution de carbonate de sodium ou d'hydroxyde de sodium. La quantité d'agent alcalin est calculée pour avoir un ratio molaire agent alcalin / H₂SO₄ supérieur ou égal à 2/1. On utilise généralement une solution aqueuse de carbonate de sodium de concentration comprise entre 5 et 10%, qui est ajoutée en continu sur une durée d'une dizaine de minutes. On observe la formation de 2 phases qui décantent dans le décanteur 40 en quelques minutes à la température de neutralisation, conduisant à une phase aqueuse 41 éliminant le sulfate acide d'alkyle neutralisé et une phase organique 50 comprenant le phtalate de dialkyle dans le solvant débarrassé du sulfate acide d'alkyle.

Après séparation de la phase aqueuse 41, on adresse ladite phase organique décantée 50 dans une colonne de distillation 60 afin d'éliminer en tête de colonne 80 le solvant et l'eau résiduelle à une température comprise entre 75°C et 110°C en abaissant progressivement la pression de service de la pression atmosphérique jusqu'à environ 20 mmHg. La présence d'un agent alcalin, de préférence de même nature que celui utilisé pour l'étape précédente de neutralisation avec une quantité sensiblement identique, permet d'éviter des problèmes de moussage au cours de la distillation effectuée dans cette étape d) du procédé selon l'invention.

La colonne 60 d'élimination du solvant peut être le siège d'une formation d'une émulsion stable généralement dans le cas où dans cette colonne la teneur en RHSO₄ et/ou RNaSO₄ est supérieure à 0,2% par rapport au mélange solvant (tel que cyclohexane) - eau, ce mélange pouvant être dans les proportions allant de 90/10 à 10/90.

La présence de RHSO₄ et/ou RNaSO₄ dans le flux alimentant la colonne 60 peut résulter d'un dysfonctionnement ou d'un contrôle insuffisamment maîtrisé de l'étape c) de neutralisation - décantation du mélange réactionnel issu de l'estérification.

L'invention remédie efficacement à tout dysfonctionnement de l'étape c) par introduction en 55 d'un additif tel que défini précédemment dans la phase organique décantée alimentant la colonne d'élimination du solvant. En cas toutefois de formation de l'émulsion, celle-ci peut être cassée, selon l'invention, en ajoutant ledit additif en tête 65 de la colonne de distillation.

On ne sortirait pas du domaine de l'invention en ajoutant également au moins une partie de l'additif dans le mélange réactionnel issu de l'estérification avant sa neutralisation, auquel cas la décantation s'en trouve facilitée.

L'étape finale e) du procédé (non représentée sur la figure) consiste en un traitement thermique sur la phase organique obtenue à l'étape d), c'est-à-dire sur le mélange brut neutralisé qui contient un agent alcalin et qui a été débarrassé du solvant. Ce traitement, généralement effectué dans des conditions de température de 180°C à 220°C et sous une pression de 15 à 20 bars, permet de dégrader en alcool, CO₂ et sulfate de sodium, le sulfate de di-alkyle issu de la réaction de l'alcool avec le catalyseur. Cette étape a une durée de l'ordre d'une heure et permet d'obtenir un phtalate de dialkyle stable thermiquement du fait de l'élimination du sulfate de di-alkyle.

Après ce traitement, le produit brut est lavé à l'eau pour l'en débarrasser de ses sels. Plusieurs cycles de lavage / séparation de la phase aqueuse peuvent être effectués, puis le produit brut est étêté par distillation pour éliminer l'eau et l'alcool résiduels et conduit à un phtalate de dialkyle de pureté > 99,5%. La distillation finale peut être effectuée à l'aide d'une colonne à distiller, d'un évaporateur rotatif, d'un évaporateur à film.

La présence finale de l'additif ne génère aucun dysfonctionnement pour le traitement des eaux générées au cours de la production du phtalate de dialkyle selon l'invention..

L'invention sera mieux comprise à la lumière des exemples non limitatifs et purement illustratifs suivants.

### Exemple 1

Dans un réacteur en verre agité mécaniquement, surmonté d'une colonne à distiller pourvue d'un condenseur de tête à eau glycolée, d'un séparateur à vide, d'un décanteur, de recettes et d'un piège à carboglace, on charge :
- 684g de -propyl heptanol (2-PH), un tel produit peut être obtenu auprès de la Sté BASF;
- 266g d'anhydride phtalique (PAA), origine Arkema

On chauffe le mélange sous agitation à 135°C sous pression atmosphérique pendant 1 heure. Le milieu, hétérogène au début, devient progressivement limpide. La première étape de réaction est terminée.

On introduit ensuite à chaud, 2,2g d'H₂SO₄ 94% dilué avec 2,2g d'eau (soit 4,4g de solution acide) et 150g de cyclohexane.

Le mélange est chauffé à pression atmosphérique. L'eau générée à la réaction est éliminée en continu par distillation sous la forme d'un hétéroazéotrope eau/cyclohexane. La température dans le mélange réactionnel est comprise entre 122°C et 135°C.

Après 5 heures de mise en réaction, le taux de conversion du PAA en DPHP est > 99%. C'est la fin de la seconde étape d'estérification.

Le brut réactionnel (BR) (1070g) est ensuite refroidi vers 60°C et neutralisé avec une solution aqueuse à 5 % de Na₂CO₃ (< 1 mole Na₂CO₃ / équivalent H⁺). La quantité molaire de Na₂CO₃ introduite est volontairement en défaut par rapport à la stoechiométrie en équivalents H⁺ présents dans le milieu afin de simuler un dysfonctionnement. La durée de la neutralisation, qui s'effectue dans le réacteur décrit ci-avant, est de 10 min environ.

Le brut est ensuite décanté à 60°C (Brut N/D) et la phase aqueuse est éliminée. On étête ensuite la phase organique en distillant l'eau et le cyclohexane présents. La distillation est conduite en faisant baisser la pression de service de la pression atmosphérique à 100 mmHg en fin d'élimination du cyclohexane. On observe dans ce cas la formation d'une émulsion stable, d'aspect crémeux, dans la colonne de distillation du cyclohexane.

L'introduction d'une petite quantité (environ 60ppm par rapport au brut N/D) de PROCHINOR FL 4809 en tête de la colonne permet de casser immédiatement cette émulsion.

La formation de cette émulsion sur l'unité industrielle imposerait son arrêt immédiat s'il n'y avait pas moyen d'y remédier.

### Exemple 2

L'exemple 1 est repris en introduisant 10 ppm de PROCHINOR FL 4809 dans le brut N/D avant la distillation du cyclohexane et de l'eau présents.

Dans ce cas, on n'observe pas la formation d'une émulsion stable malgré la présence de RHSO₄ (avec R= 2-PH) à caractère émulsionnant.

### Exemple 3

L'exemple 1 est repris en introduisant 86 ppm d'AQUAMAX EC 3379A en tête de la colonne de distillation. L'émulsion formée est immédiatement cassée.

### Exemple 4

L'exemple 2 est repris en introduisant 15 ppm d'AQUAMAX EC 3379A dans le brut N/D. On n'observe pas la formation d'émulsion dans la colonne de distillation du cyclohexane.

### Exemple 5

L'exemple 4 est repris en ajoutant volontairement 0,3% de RNaSO₄ dans le brut N/D afin de simuler une mauvaise décantation.

Dans ce cas, on observe la formation d'une émulsion stable dans la colonne de distillation du cyclohexane liée au caractère émulsionnant du RNaSO₄ (avec R=2-PH).

L'introduction d'une petite quantité de PROCHINOR FL 4809 (environ 50ppm par rapport au brut N/D) en tête de la colonne de distillation permet de casser immédiatement cette émulsion.

### Exemple 6

L'exemple 4 est repris en ajoutant volontairement 0,3% de RNaSO₄ (R= 2-PH) et 10 ppm de PROCHINOR FL 4809 dans le brut N/D.

Dans ce cas, on n'observe pas la formation d'une émulsion stable dans la colonne de distillation du cyclohexane.

### Exemple 7 (comparatif)

L'exemple 1 est repris en substituant le 2-propyl heptanol par du 2-ethyl-hexanol (2-EH) avec un rapport molaire PAA/2-EH identique à celui PAA/2-PH. Dans ce cas, on n'observe pas la formation d'une émulsion stable dans la colonne de distillation du cyclohexane malgré la présence de RHSO₄ (R=2-EH) dans le brut N/D. Ceci s'explique par le caractère peu émulsionnant du sulfate acide de 2-éthyl hexyle dans les conditions de l'essai.

### Exemple 8 (comparatif)

L'exemple 7 est repris en neutralisant le brut BR avec un excès de solution aqueuse de Na₂CO₃ à 5% (1,3 mole Na₂CO₃/équivalent H⁺), puis en ajoutant volontairement 0,3% de RNaSO₄ (R= 2- EH) dans le brut N/D.

Dans ce cas, on n'observe pas la formation d'une émulsion stable dans la colonne de distillation du cyclohexane. Ceci s'explique par le caractère peu émulsionnant du RNaSO₄ (avec R=2-EH) dans les conditions de l'essai.

### Exemple 9

L'exemple 1 est repris en introduisant 10 ppm d'un alcool polyvinylique de masse molaire 22000 ayant un taux d'hydrolyse de 99% dans le brut N/D avant la distillation du cyclohexane et de l'eau présents.

Dans ce cas, on n'observe pas la formation d'une émulsion stable malgré la présence de RHSO₄ (avec R= 2-PH) à caractère émulsionnant.

### Exemple 10

L'exemple 1 est repris en introduisant 10 ppm de polychlorure d'aluminium FLOQUAT PAC 18 contenant entre 16 et 20% d'Al₂O₃ , dans le brut N/D avant la distillation du cyclohexane et de l'eau présents.

Dans ce cas, on n'observe pas la formation d'une émulsion stable malgré la présence de RHSO₄ (avec R= 2-PH) à caractère émulsionnant.

### Exemple 11

L'exemple 1 est repris en introduisant 400 ppm de MgSO₄ , 7H₂O, dans le brut N/D avant la distillation du cyclohexane et de l'eau présents.

Dans ce cas, on n'observe pas la formation d'une émulsion stable malgré la présence de RHSO₄ (avec R= 2-PH) à caractère émulsionnant.

## Revendications

1. Procédé de fabrication de phtalate de dialkyle par estérification d'anhydride phtalique avec un alcool ROH, R représentant un groupe alkyle linéaire ou ramifié comportant de 9 à 13 atomes de carbone, de préférence de 9 à 11 atomes de carbone, en présence d'acide sulfurique et d'un solvant formant un azéotrope avec l'eau, **caractérisé en que** l'on ajoute au moins un additif choisi parmi les polymères cationiques hydrosolubles, les alcools polyvinyliques de préférence partiellement hydrolysés avec un taux d'hydrolyse allant notamment de 38 à 99%, de préférence de 70 à 90%, les polychlorures d'aluminium, ou le sulfate de magnésium anhydre ou hydraté, ledit additif étant ajouté,
soit dans la phase organique décantée après neutralisation du mélange réactionnel issu de l'estérification,
soit en tête de la colonne de distillation finale du solvant présent dans la phase organique décantée après neutralisation du mélange réactionnel issu de l'estérification.

2. Procédé de fabrication de phtalate de dialkyle par estérification d'anhydride phtalique avec un alcool ROH, R représentant un groupe alkyle linéaire ou ramifié comportant de 9 à 13 atomes de carbone, de préférence de 9 à 11 atomes de carbone, comprenant les étapes suivantes :
a) on met à réagir un alcool ROH avec de l'anhydride phtalique dans un rapport molaire alcool / anhydride allant de 2,1 à 2,4, à une température allant de 120°C à 140°C, en l'absence de catalyseur, jusqu'à l'obtention d'une phase homogène limpide,
b) on ajoute à chaud de l'acide sulfurique à une teneur comprise entre 0,2% à 0,9% (exprimé en H₂SO₄ 100%), par rapport à l'anhydride phtalique, et un solvant organique, la température de réaction étant maintenue entre 120°C et 140°C et l'eau étant éliminée en continu par distillation sous forme d'un hétéroazéotrope avec le solvant, durant toute la durée de la réaction,
c) on neutralise le mélange brut réactionnel à l'aide d'un agent alcalin à une température comprise entre 50°C et 70°C, et on sépare, après décantation, une phase organique et une phase aqueuse,
d) on distille le solvant de ladite phase organique,
e) on effectue un traitement thermique de la phase organique obtenue à l'étape d), puis différents cycles de lavages à l'eau/décantation, et une distillation finale pour obtenir le phtalate de dialkyle pur recherché,
**caractérisé en que** l'on ajoute au moins un additif choisi parmi les polymères cationiques hydrosolubles, les alcools polyvinyliques de préférence partiellement hydrolysés avec un taux d'hydrolyse allant notamment de 38 à 99%, de préférence de 70 à 90% les polychlorures d'aluminium, ou le sulfate de magnésium anhydre ou hydraté, soit à l'étape c) dans la phase organique après décantation, soit au cours de l'étape d) en tête de la colonne de distillation.

3. Procédé selon la revendication 1 ou 2 **caractérisé en ce que** l'alcool ROH est le décanol, le 2-propyl heptanol, le tridécanol, l'isononanol, l'isodécanol, le dodécanol, l'undécanol.

4. Procédé selon l'une quelconque des revendications précédentes **caractérisé en ce que** l'alcool est 2-propyl heptanol.

5. Procédé selon la revendication 4 **caractérisé en ce que** l'alcool est un mélange comprenant :
- de 70 à 99%, de préference de 80 à 95% de 2-propyl heptanol n-C₅H₁₁CH(C₃H₇)CH₂OH et
- de 1 à 30%, de préférence de 5 à 20% d'un mélange de 4-méthyl 2-propyl hexanol C₂H₅CH(CH₃)CH₂CH(C₃H₇)CH₂OH et de 5-méthyl 2-propyl hexanol CH₃CH(CH₃)CH₂CH₂CH(C₃H₇)CH₂OH .

6. Procédé selon l'une quelconque des revendications précédentes **caractérisé en ce que** le solvant est le cyclohexane.
